# EUROPEAN PATENT APPLICATION

(11) **EP 1 746 082 A1**
(43) Date of publication of application: **24.01.2007**
(21) Application number: 05015584.5
(22) Date of filing: 19.07.2005
(51) Int. Cl.: C07C 53/16, C07C 53/18, C07C 53/19, C07C 53/21, A61K 31/315, A61K 31/19, A61P 17/00

(54) **Salts of zinc and aliphatic haloid carboxylic acids for therapy of skin neoplasms and visible mucous coats**

(71) Applicant: Tsyb, Anatoly Fyodorovich, Obninsk 249020 (RU); Mardi, Shalva, 4102 Binningen (CH); Krikunova, Lyudmila Ivanovna, Obninsk 249039 (RU)
(72) Inventor: Tsyb, Anatoly Fyodorovich, Obninsk 249020 (RU); Mardi, Shalva, 4102 Binningen (CH); Krikunova, Lyudmila Ivanovna, Obninsk 249039 (RU)
(74) Representative: von Füner, Nicolai

(57) **Abstract**

The invention relates to medicine, and more particularly to dermatology, namely to new salts of zinc and aliphatic haloid carboxylic acids which can be used to treat benign skin lesions and visible mucous coats.

The following chemical formula of salts of zinc and aliphatic haloid carboxylic acids is proposed 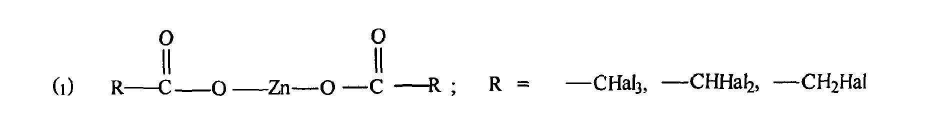 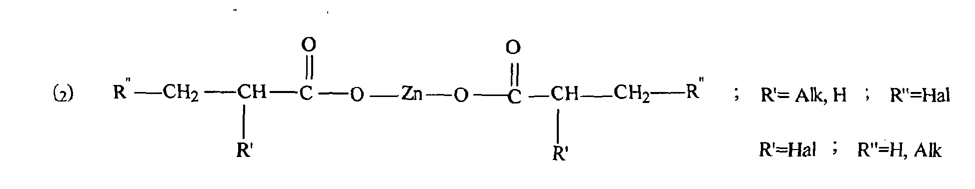 wherein in formulae Fluorine (F), Chlorine (Cl), Bromine (Br) or Iodine (J) can be a halogen atom.

The obtained technical result is the creation of a unique preparation to treat benign skin lesions and visible mucous coats. The preparation is low-toxic, fast acting with a pronounced therapeutic effect and a good tolerance. It causes no complications during therapy, and ensures healing without scar tissue formation. The created preparation allows to extend the assortment of drugs for therapy of similar diseases.

## Description

The pesent invention relates to medicine, and more particularly to dermatology, namely to new salts of zinc and aliphatic haloid carboxylic acids which can be used to treat benign skin lesions and visible mucous coats.

A compound of selenium with gallic-acetic acid or its anhydride (1) is known. The compound is intended for therapy of benign skin neoplasms of different nature including mucous coats.

It is also known that prospidinum ointment with low toxicity is used to treat papillomatosis of mucous membranes of upper airways (2). In this case, the therapeutic effectiveness is 70 - 75 %. Prospidinum appears to be the most effective in the treatment of premalignant lesions.

Taking into account marked deep tissue lesions, it is not advisable to use prospidinum to treat benign skin lesions and mucous coats.

The best way to achieve the aimed effect is to use the preparation for the local treatment of superficial skin and mucous membrane lesions on the basis of interaction of 1-5,5 M nitric acid solution and 45-170 mmoles of primary C₁-C₅-alcanol for 1 liter nitric acid (3-prototype). In particular, the preparation is useful for the local treatment of superficial benign skin lesions, such as verrucae (for example, verrucae vulgares and verrucae plantares), seborrheic and actinic keratosis and condylomata as well as superficial benign lesions of mucous membrane, for example benign cervical lesions, such as erosions, ovula nabothi polyps in the cervical canal, condylomata.

However, the use of the preparation results in the recurrence rate of 30 - 40% and incomplete healing of 45 %. The preparation is a certain hazard to the personnel and caution should be exercised in handling it.

The purpose of the invention is to create a unique low-toxic stable highly effective preparation for therapy of benign skin neoplasms and visible mucous coats having a good tolerance, no complications during treatment and ensuring healing without scar tissue formations.

The following chemical formula of salts of zinc and aliphatic haloid carboxylic acids is proposed 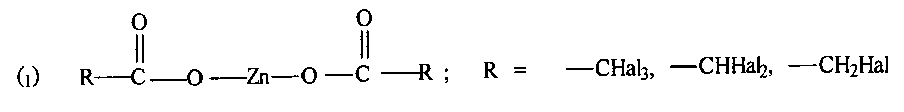 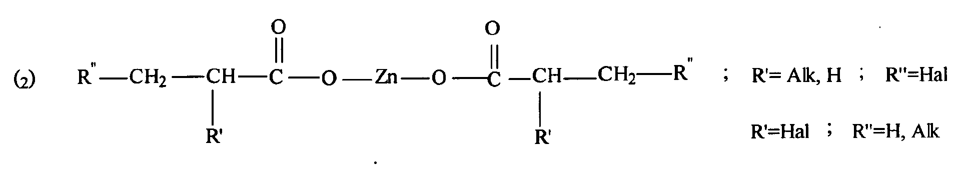 wherein in formulae Fluorine (F), Chlorine (Cl), Bromine (Br) or Iodine (J) can be a halogen atom.

As initial materials can be various chemical products containing zinc and solutions of aliphatic haloid carboxylic acids.

The end product is a solution of salt of zinc and haloid carboxylic acids. It is brown, pH = 2.2-3.0, LD50 = about 1100 mg/kg. The preparation is practically non-toxic, it does not penetrate into the body and it shows no system, teratogenic, embryotoxic or cancerogenic properties. The preparation selectively dehydrates the tissues, it has an intravital fixing effect on the tissues. Two weeks after excision of the abnormal tissues, healing and regeneration occur without significant side effects.

The zinc concentration in the product depends on the type of initial chemical ingredients and can range between 2,5-25 %. Chemically, the product is unchangeable for many years during storage at room temperature in a closed dark glass vessel.

The claimed substance can be used only for local application. As drug, it can be in the form of a solution in a concentration of 5-45%. The recommended dose for one 0,1-2,0 cm² lesion in liquid form is from 0,03 up to 0.2 ml. In the case of numerous lesions, the dose can be from 0,2 up to 2,00 ml.

The obtained technical result is the creation of a unique preparation for therapy of benign skin lesions and visible mucous coats. The preparation is low-toxic, fast acting with a pronounced therapeutic effect and a good tolerance. It causes no complications during therapy, and ensures healing without scar tissue formation. The created preparation allows to expand the assortment of preparations for therapy of similar diseases.

The preparation is easy to use, safe for patients and medical staff, and it does not require special restrictions of the behaviour mode.

From the patent and scientific-and-technical information, the chemical formula of salts of zinc and aliphatic haloid carboxylic acids which could be used for local treatment of benign skin neoplasms and visible mucous coats is not known.

The synthesis of the claimed substance is based on the use of accessible and inexpensive raw material and is easy.

The claimed substance is obtained through a reaction of oxide, or hydroxide, or zinc carbonate with haloid carboxylic acids in the water medium and stabilization of obtained solutions by the addition of 0,72 - 1,36 % of salts of amber or citric acids. 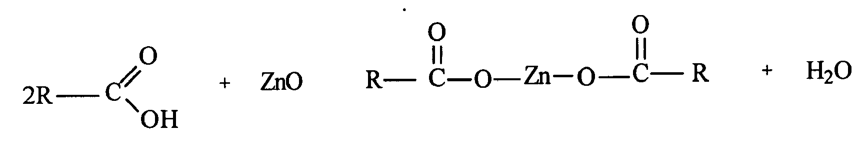 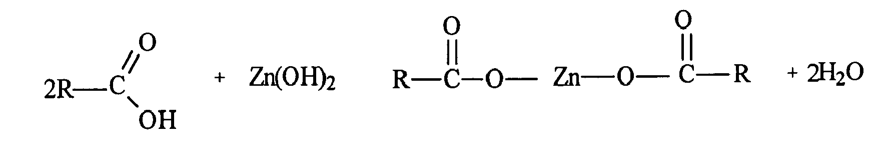 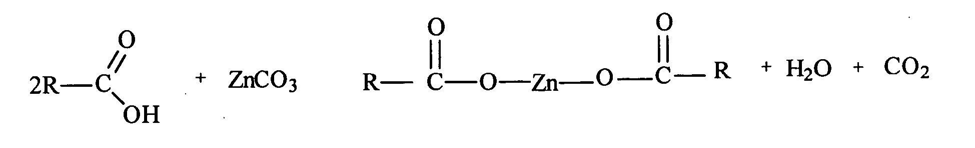 The structure of the new substance is confirmed by the results of the elementary analysis.

### Example 1. Zinc di-(bromacetate).

To suspension of 2,44 g (0,03 mole) zinc oxide in 30 ml distilled water 8,34 g (0,06 mole) bromoacetic acid are added in one step. The reaction mixture is mixed to the almost full sediment dissolution (for about 15 min) at room temperature, a minor amount of mixture is filtered through a paper filter using the Büchner funnel. 32 ml of 23,8% solution of bromoacetate and zinc salt are produced. After lyophylization of aliquot solution, dry salt is produced and its elementary composition is determined. C4H4Br2O4Zn. In order to stabilize solution, 0,3 g amber acid is added to it.
It is calculated, %: C 14,06; H 1,17; Br 46,87; Zn 19,16.
It is found, %: C 13,95; H 1,12; Br 46,55; Zn 19,24.

### Example 2. Zinc di-(trichloroacetate).

It is produced after Example 1 from 2,44 g (0,03 mole) zinc oxide, 9,81 g (0,06 mole) trichloroacetic acid and 20 ml distilled water. The concentration of produced salt is 36,3%. C4C16O4Zn. In order to stabilize solution, 0,3 g citric acid are added to it.
It is calculated, %: C 12,30; Cl 54,56; Zn 16,75.
It is found, %: C 12,39; Cl 54,47; Zn 16,62.

### Example 3. Zinc di-(chloroacetate).

To suspension of zinc hydroxide obtained from 4,1 g (0,03 mole) zinc chloride and 2,4 g (0,06 mole) sodium hydroxide, solution of 5,67 g (0,06 mole) chloroacetic acid in 25 ml distilled water is added. It is mixed at room temperature to an almost full resolution of precipitate, is filtered through a paper filter using the Büchner funnel to produce 22,5 % solution of chloroacetate and zinc salt. After lyophylization of aliquot solution, dry salt is produced and its elementary composition is determined. C₄H₄Cl₂O₄Zn. The solution is stabilized using 0,3 g amber acid.
It is calculated, %: C 19,02; H 1,59; Cl 28,13; Zn 25,91.
It is found, %: C 19,89; H 1,63; Cl 28,01; Zn 26,03.

### Example 4. Zinc di-(2-chloropropionate).

It is produced after Example 3 from 4,1 g (0,03 mole) chloride zinc, 2,4 g (0,06 mole) sodium hydroxide with subsequent processing of zinc hydrooxyde using solution of 6,5 g (0,06 mole) 2-chloropropione acid in 20 ml distilled water. The concentration of produced salt is 28,5 %. C₆H₈Cl₂O₄Zn. The solution is stabilized using 0,3 g citric acid.
It is calculated, %: C 25,68; H 2,85; Cl 25,32; Zn 23,32.
It is found, %: C 25,75; H 2,71; Cl 25,42; Zn 23,27.

### Example 5. Zinc di-(2-methyl-3-brompropionate).

It is produced after Example 1 from 2,44 g (0,03 mole) zinc oxyde, 10 g (0,06 mole) 2-methyl-3-brompropione acid and 25 ml distilled water. The concentration of produced salt is 30,3 %. C₈H₁₂Br₂O₄Zn. The solution is stabilized using 0,3 g amber acid.
It is calculated, %: C 24,16; H 3,02; Br 40,26; Zn 16,46.
It is found, %: C 24,03; H 3,15; Br 40,37; Zn 16,38.

### Example 6. Zinc di-(3-brompropionate).

To suspension of 3,76 g (0,03 mole) zinc carbonate in 30 ml distilled water 9,18 g (0,06 mole) 3-brompropione acid are added in one step while stirring. The reaction mixture is mixed till full termination of carbonic gas evolution, sediment is filtered through a paper filter using the Büchner funnel. 25,9% solution of salt of 3-brompropione acid and zinc is obtained. After lyophylization of aliquot solution, dry salt is obtained and its elementary composition is studied. C₆H₈Br₂O₄Zn. The solution is stabilized using 0,3 g citric acid.
It is calculated, %: C 19,49; H 2,17; Br 43,31; Zn 17,71.
It is found, %: C 19,58; H 2,29; Br 43,22; Zn 17,62.

### Example 7. Zinc di-(2,3-dibrompropionate).

It is produced after Example 6 from 3,76 g (0,03 mole) zinc carbonate, 13,92 g (0,06 mole) 2,3-dibrompropione acid and 25 ml distilled water. The concentration of produced salt is 36,9%. C₆H₆Br₄Zn. The solution is stabilized using 0,3 g amber acid.
It is calculated, %: C 13,65; H 1,14; Br 60,68; Zn 12,40.
It is found, %: C 13,53; H 1,19; Br 60,54; Zn 12,51.

### Example 8. Zinc di-(...-iodoacetate).

It is produced after Example 1 from 2,44 g (0,03 mole) zinc oxyde, 12 g (0,06 mole) ..-iodoacetic acid and 30 ml distilled water. The concentration of produced salt is 30,1 %. C₄H₄J₂O₄Zn. The solution is stabilized using 0,3 g citric acid.
It is calculated, %: C 11,02; H 0,92; J 58,34; Zn 15,02.
It is found, %: C 10,91; H 1,01; J 58,01; Zn15,14.

### Example 9. Zinc di-(trifluoroacetate).

It is produced after Example 1 from 2,44 g (0,03 mole), 6,84 g (0,06 mole) trifluoroacetic acid and 15 ml distilled water. The concentration of produced salt is 33,8 %. C₄F₆O₄Zn. ...
It is calculated, %: C 16,47; F 39,12; Zn 22,44.
It is found, %: C16,56; F 39,24; Zn 22,32.

### Example 10. Zinc di-(fluoroacetate).

It is produced after Example 6 from 3,76 (0,03 mole) zinc carbonate, 4,68 g (0,06 mole) fluoroacetic acid and 15 ml distilled water. The concentration of produced salt is 27,3 %. C₄H₄F₂O₄Zn. ....
It is calculated, %: C 21,88; H 1,82; F 17,32; Zn 29,81.
It is found, %: C 21,82; H 1,73; F 17,44; Zn 29,69.

### Example 11. Zinc di-(dichloracetate).

It is produced after Example 1 from 2,44 g (0,03 mole) zinc oxyde, 7,74 g (0,06 mole) dichloroacetic acid and 20 ml distilled water. The concentration of produced salt is 30,2 %. C₄H₂Cl₄O₄Zn. ....
It is calculated, %: C 14,94; H 0,62; Cl 44,18; Zn 20,35.
It is found, %: C14,83; H 0,57; Cl 44,27; Zn 20,00.

### Example 12. Treatment of 11 patients with ectopia of cervix uteri (pseudo-erosion), who had recurrent pseudo-erosion (age from 26 to 32 years).

30% solution of zinc salt of haloid carboxylic acid was used to treat ectopias. Depending on the extent of the pathological focus, the number of preparation applications was different. In ectopias up to 0,5-0,8 mm in diameter, processing was performed one time within 2 days. In larger lesions, double application was used one time, then, every day or every two days. In total, 3-4 applications were performed. The preparation was administered using cotton-wool tampons as application on the zone of lesion. The immediate effect manifested as whitish-yellow tissue stain with a clear white ischemic elevation around the zone of lesion; on the 2nd - 3rd day, the tissue became gray-white; from the 3rd day, the crust decreased and tore away. In place of the former crust, cervical mucosa got a reddish tint. Epithelization lasted from 10 to 14 days. During this period, regeneration with young epithelium of cervical mucosa occurred. In extensive cervical lesions requiring a deep tissue destruction, the duration of epithelization increased up to 3 - 5 weeks. As side effects, slight burning was noted at the moment of processing.

At 3-4 days after last processing, control inspection revealed the crust rejection and the presence of reddish-crimson cervical mucosa practically in all the cases.

Two weeks later, control inspection revealed that cervical mucosa got a rose-red tint, i.e. it got more normal color. Colpocopically, metaplastic (young) epithelium through the whole surface, i.e. epithelization process was seen. Cytological investigation revealed a stratified flat epithelium.

Patients with ectopias were followed-up from 12 days to 2 months. During this period of follow-up, only one (9,1 %) patient with prior extensive eroded surface of the cervix did not show a full mucosa epithelization and probably because of the lesion depth.

Thus, 90% of patients with cervical ectopias (background diseases) were found to have significant benefit from solution of zinc salt of haloid carboxylic acids.

### Example 13. Treatment of 7 patients with pointed condyloma of papilloviral infection (papilloma) (age from 20 to 68 years).

30% solution of zinc salt of haloid carboxylic acids was used to treat patients with papillomas. Every day, 3-4 applications and in large papillomas up to 6 applications were performed. Directly during processing, reduction of mass volume (shrinking), i.e. dehydration, mummification with formation of a whitish-yellow incrustation was observed. Before each subsequent processing, necrotic tissues were removed. After gradual rejection, dark-bluish-brown spots remained on skin within 7-14 days.

Cytologically, flat epithelium without signs of pathology was noted after the completion of treatment.

At 2 months, of 7 patients with papillomas, one patient with prior papilloma sized not less than 4 cm in diameter developed a recurrence.

Thus, the primary effect in the treatment of papillomas is 100 %.

As follows from examples, the preparation of zinc salt and haloid carboxylic acids has beneficial therapeutic effects on the healing of cervical ectopias and papillomatous skin lesions of genital organs.

The preparation includes the following advantages: good tolerance, absence of complications, healing without scar tissue formation, easy performance, safety for patients and medical staff as well as absence of special restrictions of the behaviour mode.

Thus, the claimed chemical substance - salts of zinc and aliphatic haloid carboxylic acids - is especially beneficial in the treatment of skin neoplasms and visible mucous coats.

### Information sources

1. EP 1293498 A1, MKH: C07C391/00, priority of 30.08.2001.
2. Prospidinum - a new antineoplastic agent. VNIIKhFI, issue 3, Moscow, 1973, p.115-138.
3. RU No. 2118160, MKH: A 61 K 31/19, priority of 23.06.93 - prototype.

## Claims

1. FORMULA OF THE INVENTION
Salts of zinc and aliphatic haloid carboxylic acids 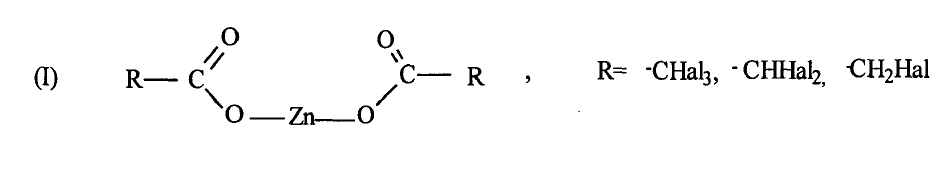 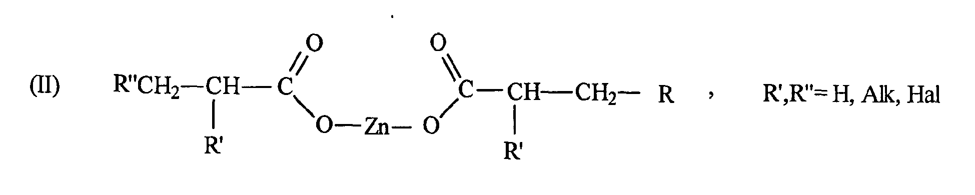 wherein in formulae Fluorine (F), Chlorine (Cl), Bromine (Br) or Iodine (J) can be a halogen atom.
